# EUROPÄISCHE PATENTSCHRIFT

(11) **EP 2 001 417 B1**
(45) Veröffentlichungstag und Bekanntmachung des Hinweises auf die Patenterteilung: **15.05.2013**
(21) Anmeldenummer: 07723622.2
(22) Anmeldetag: 27.03.2007
(51) Int. Cl.: A61F 2/44, A61F 2/48

(54) **ZWISCHENWIRBELIMPLANTAT**
INTERVERTEBRAL IMPLANT
IMPLANT INTERVERTÉBRAL

(30) Priorität: 06.04.2006 DE 102006016987
(43) Veröffentlichungstag der Anmeldung: 17.12.2008
(73) Patentinhaber: Aesculap AG, 78532 Tuttlingen (DE)
(72) Erfinder: BEGER, Jens, 78532 Tuttlingen (DE)
(74) Vertreter: Hoeger, Stellrecht & Partner Patentanwälte
(86) Internationale Anmeldenummer: PCT/EP2007/002676
(87) Internationale Veröffentlichungsnummer: WO 2007/115677

(56) Entgegenhaltungen:
- WO-A-2004/103226
- WO-A-2007/040708
- US-A1- 2004 167 625
- US-A1- 2005 256 576

## Beschreibung

Die Erfindung betrifft ein Zwischenwirbelimplantat mit mindestens einem eine Wirbelkörperanlagefläche ausbildenden Anlageelement, bei dem die Wirbelkörperanlagefläche mindestens zwei relativ zueinander bewegliche Teile umfasst, die in einer ersten Einführposition relativ zueinander so angeordnet sind, dass sie gemeinsam einen kleinen Querschnitt einnehmen, und in einer zweiten Implantierposition so, dass der Querschnitt der Wirbelkörperanlagefläche gegenüber der Einführposition vergrößert ist, und mit einer Verstellvorrichtung zur Bewegung der beweglichen Teile von der Einführposition in die Implantierposition, wobei die Verstellvorrichtung mindestens ein flexibles Zugelement umfasst, welches an einem der relativ zueinander beweglichen Teile angreift und auf Zug dieses relativ zum anderen Teil in die Implantierposition bewegt.

Zwischenwirbelimplantate sollten eine möglichst große Wirbelkörperanlagefläche aufweisen, die der Größe der Wirbelkörperendflächen angepasst ist, an denen das Zwischenwirbelimplantat zur Anlage kommt. Dadurch wird die Gefahr eines Einbrechens oder Einsinterns des Zwischenwirbelimplantates in den Wirbelkörper herabgesetzt.

Andererseits ist es wünschenswert, das Implantat durch einen möglichst kleinen Zugang in den Zwischenwirbelraum zu bringen, insbesondere beim Einführen des Implantates durch einen posterioren, transforaminalen oder lateralen Zugang.

Es ist bekannt, die Anlageflächen von Zwischenwirbelimplantaten zu diesem Zweck mehrteilig auszubilden und die verschiedenen Teile in einer eingeklappten Stellung in den Körper einzuführen. Nach dem Einführen in den Körper durch einen relativ kleinen Zugang können die beweglichen Teile in eine aufgeklappte Stellung verbracht werden, in der eine relativ große Wirbelkörperanlagefläche ausgebildet wird, deren Querschnitt deutlich größer ist als der Querschnitt der Zugangsöffnung in den Körper (W02004/103226A2). Um bei dieser bekannten Vorrichtung die beweglichen Teile in die ausgeklappte Implantierposition zu verbringen, sind entweder komplizierte Verstelleinrichtungen mit Gewindespindeln notwendig, oder aber dieses Ausklappen muss durch spezielle Instrumente erfolgen, die der Arzt durch den Zugang in den Körper einführen muss und mit denen er die Bewegung der beweglichen Teile vornehmen muss. Dies ist bei den relativ kleinen Zugängen außerordentlich schwierig und auch gefährlich, da beim Aufklappen gegebenenfalls relativ große Kräfte übertragen werden müssen und da verletzliche Körperteile in unmittelbarer Umgebung des Zwischenwirbelraums angeordnet sind.

Es ist auch bereits vorgeschlagen worden, zur Verstellung der beweglichen Teile ein flexibles Zugelement in Form eines Fadens zu verwenden (WO2007/040708). Dieses Dokument fällt unter Art. 54 (3) EPÜ und offenbart ein Zwischenwirbelimplantat, das sich von der vorliegenden Erfindung dadurch unterscheidet, dass auf der der Wirbelkörperanlagefläche abgewandten Seite des Anlageelements quellfähiges Material angeordnet ist, das bei Flüssigkeitsaufnahme eine Volumenvergrößerung erfährt und dadurch die Teile des Anlageelements in die Implantierposition drückt.

Es ist Aufgabe der Erfindung, ein gattungsgemäßes Zwischenwirbelimplantat so auszubilden, dass einerseits die Bewegung der beweglichen Teile von der Einführposition in die Implantierposition vereinfacht wird und andererseits eine gewisse Beweglichkeit der beiden Anlageelemente relativ zueinander zu ermöglichen.

Diese Aufgabe wird bei dem Zwischenwirbelimplantat der eingangs beschriebenen Art erfindungsgemäß dadurch gelöst, dass auf der der Wirbelkörperanlagefläche abgewandten Seite des Anlageelements quellfähiges Material angeordnet ist, das bei Flüssigkeitsaufnahme eine Volumenvergrößerung erfährt und dadurch die Teile des Anlageelementes in die Implantierposition drückt. Es genügt also ein Ziehen an diesem flexiblen Zugelement, das fadenähnlich ausgebildet sein kann, um die Aufklappbewegung durchzuführen, dabei können ohne weiteres relativ große Kräfte übertragen werden, andererseits wird praktisch kein zusätzliches Bauvolumen benötigt, und eine Verletzungsgefahr in der Umgebung der Operationsstelle scheidet aus. Das quellfähige Material hat die Funktion eines Polsters zwischen den Anlageelementen, welches eine gewisse Beweglichkeit der beiden Anlageelemente relativ zueinander ermöglicht und dessen Rückstellkräfte unter anderem durch das Quellverhalten mitbestimmt werden, wie dies auch bei einer natürlichen Bandscheibe der Fall ist. Günstig ist es, wenn das flexible Zugelement so lang ist, daß ein Ende nach dem Einführen des Zwischenwirbelimplantat in den Körper außerhalb des Körpers verbleibt, so daß über dieses extrakorporale Ende ein Zug auf das flexible Zugelement ausgeübt werden kann. Der Operateur kann also außerhalb des Körpers das freie Ende des flexiblen Zugelements ergreifen und von dort aus durch Zug die Bewegung der beweglichen Teile in die Implantierposition veranlassen.

Das flexible Zugelement kann am anderen Teil verschieblich geführt sein, beispielsweise durch Ösen oder andere an sich bekannte Fadenführer.

Besonders vorteilhaft ist es, wenn das flexible Zugelement am anderen Teil durch eine Umlenkung geführt ist, die die Richtung des flexiblen Zugelements ändert. Dadurch ist es möglich, die Zugkraft in der gewünschten Richtung auf das bewegliche Teil zu übertragen, trotzdem kann das freie Ende des Zugelements benutzerfreundlich in Richtung der Einführöffnung des Körpers verlaufen. Zugkräfte können auf diese Weise auch in Richtungen übertragen werden, in denen infolge des engen Zugangs eine Krafteinwirkung mittels eines Instruments unmöglich wäre.

Nach der Operation kann das flexible Zugelement beispielsweise durch Abschneiden vom Implantat gelöst werden. Bei einer bevorzugten Ausführungsform ist jedoch vorgesehen, daß das flexible Zugelement lösbar mit dem durch Zug bewegten Teil verbunden ist, wobei die Lösung erst nach Überschreiten eines bestimmten Zugwertes eintritt. Mittels einer Sollbruchstelle kann zunächst die Kraft ohne weiteres übertragen werden, die zum Bewegen des beweglichen Teils in die Implantierposition notwendig ist, sobald dies erreicht ist, kann der Operateur durch Vergrößerung der Zugkraft das flexible Zugelement von dem bewegten Teil lösen und aus dem Körper entfernen.

Das flexible Zugelement kann beispielsweise ein chirurgischer Nähfaden sein.

Besonders vorteilhaft ist es, wenn das flexible Zugelement aus einem im Körper resorbierbaren Material besteht, in einem solchen Fall kann das Zugelement gegebenenfalls auch im Körper verbleiben.

An einem bewegbaren Teil können mehrere Zugelemente angreifen. Diese werden dann bevorzugt als Fadenbündel gemeinsam aus dem Operationszugang nach außen geführt.

Es kann vorgesehen sein, daß ein Anlageelement mehrere bewegbare Teile aufweist und daß jedem bewegbaren Teil mindestens ein Zugelement zugeordnet ist. Auch in diesem Fall kann der Operateur durch Zug an einem Fadenbündel gleichzeitig mehrere Zugelemente betätigen und dadurch gleichzeitig mehrere bewegbare Teile in die Implantierposition bewegen.

Bei einer bevorzugten Ausführungsform ist eine Rastvorrichtung vorgesehen, die beim Bewegen des bewegbaren Teils wirksam wird, sobald das bewegbare Teil die Implantierposition erreicht hat, und die das bewegbare Teil gegenüber dem anderen Teil fixiert. Sobald die Implantierposition durch Zug an dem flexiblen Zugelement erreicht ist, greift die Rastvorrichtung ein und verhindert eine Rückbewegung des bewegbaren Teils in die Einführposition. Der Operateur kann in diesem Augenblick die Zugkraft auf das flexible Zugelement beenden, die bewegbaren Teile des Anlageelementes verbleiben trotzdem in der einmal erreichten Implantierposition.

Beispielsweise kann die Rastvorrichtung eine mit Rasten versehene Rastfläche an einem der beiden Teile und ein elastisch gegen die Rastfläche gedrücktes Rastglied am anderen der beiden Teile aufweisen, die beim Bewegen der Teile in die Implantierposition aneinander entlang gleiten. Dabei ist eine Relativbewegung in einer Richtung ohne weiteres möglich, in der entgegengesetzten Richtung aber durch das Eingreifen des Rastgliedes in die Rasten der Rastfläche verhindert.

Bei einer anderen Ausgestaltung kann vorgesehen sein, daß die Rastvorrichtung an einem der beiden Teile gelagerte, elastisch verschiebbare Raststifte umfaßt, die in der Implantierposition federnd und formschlüssig in eine Ausnehmung am anderen der beiden Teile eingreifen.

Eine Festlegung der bewegbaren Teile in der Implantierposition kann auch dadurch erreicht werden, daß an einem Teil der beiden Teile bewegbare Stützglieder angeordnet sind, die in eine das andere der beiden Teile unterstützende Position bewegbar sind, sobald das andere der beiden Teile die Implantierposition erreicht hat.

Insbesondere kann das Stützglied mindestens ein drehbar an einem der beiden Teile gelagerter Hebelarm sein, der in eine das andere der beiden Teile unterstützende Position verdrehbar ist.

Es ist dabei vorteilhaft, wenn zum Verdrehen des Hebelarmes eine Gewindespindel an dem den Hebelarm lagernden Teil angeordnet ist.

Die vorstehend beschriebenen Rastvorrichtungen und Einrichtungen zur Festlegung der bewegbaren Teile in der Implantierposition können auch bei Zwischenwirbelimplantaten Verwendung finden, bei denen die beweglichen Teile nicht durch flexible Zugelemente in die Implantierposition gebracht werden, sondern in anderer Weise, beispielsweise mittels eingeführter Instrumente, mittels gefüllter Schwellkörper oder mittels eines quellfähigen Materials, das durch Flüssigkeitsaufnahme und Volumenvergrößerung die Bewegung der beweglichen Teile in die Implantierposition auslöst.

Es ist günstig, wenn mindestens ein Anschlag vorgesehen ist, der die Bewegung des bewegbaren Teils in die Implantierposition begrenzt. Damit ist die Implantierposition genau definiert, der Operateur muß also nicht genau überprüfen, welche Position die bewegbaren Teile einnehmen, sondern er kann so lange ziehen, bis die Implantierposition erreicht ist.

Die beweglichen Teile des Anlageelements können sehr unterschiedlich ausgebildet sein, dazu wird unter anderem Bezug genommen auf die unterschiedlichen Ausgestaltungen, die in WO2004/103226A2 beschrieben sind.

So kann beispielsweise das Anlageelement mindestens zwei plattenförmige Anlageglieder aufweisen, die relativ zueinander verschwenkbar sind und die zur Überführung in die Implantierposition auseinandergeschwenkt werden.

Das Zwischenwirbelimplantat umfaßt mindestens ein derartiges Anlageelement, vorzugsweise ist jedoch am oberen und am unteren Ende des Zwischenwirbelimplantates je ein in seiner Querschnittsgröße veränderbaren Anlageelement dieser Art angeordnet.

Erfindungsgemäß ist auf der der Wirbelkörperanlagefläche abgewandten Seite des Anlageelements quellfähiges Material angeordnet, das bei Flüssigkeitsaufnahme eine Volumenvergrößerung erfährt und dadurch die Teile des Anlageelementes in die Implantierposition drückt. Dadurch werden die bewegbaren Teile in der Implantierposition fixiert, diese Fixierung kann entweder allein vorgesehen werden oder zur Unterstützung einer Rastvorrichtung, die die bewegbaren Teile in der Implantierposition hält. Bei Verwendung eines quellfähigen Materials dieser Art ist allerdings zu bedenken, dass die Volumenvergrößerung durch Flüssigkeitsaufnahme, die bei der Implantierung eintritt, mehrere Stunden dauern kann, so dass es günstig ist, die Haltekräfte des quellfähigen Materials zu kombinieren mit einer solchen Rastvorrichtung, die zumindest beim Beginn des Quellvorgangs die Fixierung der bewegbaren Teile in der Implantierposition übernimmt.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Zwischenwirbelimplantates auf einem Wirbelkörper mit den beweglichen Teilen in der Einführposition;
- Figur 2:: eine Ansicht ähnlich Figur 1 mit den beweglichen Teilen in der Implantierposition und dem Zwischenwirbelimplantat zwischen zwei Wirbelkörpern;
- Figur 3:: eine Seitenansicht des Implantates der Figur 1 in der Einführposition;
- Figur 4:: eine Vorderansicht des Implantates in der Einführposition ;
- Figur 5:: eine Draufsicht auf das Implantat der Figur 1 in der Implantierposition;
- Figur 6:: eine perspektivische Ansicht der zwei Komponenten eines Zwischenwirbelimplantates mit einer Hebelanordnung zur Fixierung der bewegbaren Teile in der Implantierposition;
- Figur 7:: eine Schnittansicht längs Linie 7-7 in Figur 6;
- Figur 8:: eine Ansicht ähnlich Figur 7 bei einem abgewandelten Ausführungsbeispiel mit einem einarmigen Hebel als Stützglied;
- Figur 9:: eine Teilschnittansicht längs Linie 9-9 in Figur 10 des Scharnierbereichs eines Zwischenwirbelimplantats mit einer Rastvorrichtung zur Festlegung der bewegbaren Teile in der Implantierposition;
- Figur 10:: eine Teilschnitt-Draufsicht auf den Scharnierbereich des Zwischenwirbelimplantats der Figur 9 und
- Figur 11:: eine Ansicht ähnlich Figur 5 mit einer Rastvorrichtung mit Raststiften zur Festlegung der bewegbaren Teile in der Implantierposition.

Das in der Zeichnung dargestellte Zwischenwirbelimplantat 1 wird beim Implantieren in den Zwischenwirbelraum 2 zwischen zwei Wirbelkörper 3, 4 eingeschoben und ersetzt dort die aus dem Zwischenwirbelraum 2 entfernte Bandscheibe. Das in der Zeichnung dargestellte Zwischenwirbelimplantat 1 umfaßt an seiner Unterseite und an seiner Oberseite je ein Anlageelement 5 beziehungsweise 6, die beide gleich aufgebaut, jedoch spiegelbildlich zueinander angeordnet sind. Nachstehend wird nur eines der beiden Anlageelemente näher erläutert.

Das Anlageelement 5 weist einen mittleren, plattenförmigen, im Querschnitt rechteckigen Stützabschnitt 7 auf, an dessen Längsseiten um eine an den Längskanten entlang laufende Schwenkachse jeweils ein plattenförmiges Schwenkteil 8, 9 gelagert ist. Das eine Schwenkteil hat die Form eines Kreissektors mit einer bogenförmigen äußeren Kante 10, das andere Schwenkteil 9 ist im wesentlichen rechteckig ausgebildet, jedoch ist die der Schwenkachse abgewandte äußere Kante 11 geringfügig nach innen gebogen und geht an ihren Enden über Abrundungen 12 in die quer verlaufenden Kanten 13, 14 über. Wenn die beiden Schwenkteile 8, 9 in die Ebene des Stützabschnitts 7 geschwenkt sind, ergibt sich auf diese Weise an der Außenseite des jeweiligen Anlageelementes 5 eine plane Anlagefläche 15, die sich aus den Einzelflächen des Stützabschnitts 7 und der beiden Schwenkteile 8, 9 zusammensetzt und die auf gegenüberliegenden Längsseiten in gleicher Richtung bogenförmig begrenzt ist. Diese Anlagefläche 15 ist damit an die Kontur der Endflächen der beiden Wirbelkörper 3, 4 angepaßt und kann so groß gewählt werden, daß sie im wesentlichen an der gesamten Stirnfläche der Wirbelkörper 3, 4 anliegt.

Bei einer abgewandelten Ausführungsform könnten die beiden Schwenkteile 8, 9 auch gegenüber dem zentralen Stützabschnitt 7 geringfügig geneigt sein, so daß eine optimale Anpassung an die jeweilige Geometrie der Wirbelstirnfläche erzielt werden kann. In diesem Falle ergibt sich eine Anlagefläche des Implantates, die nicht im gesamten Bereich plan ist, sondern die Bereiche mit geringfügig unterschiedlicher Neigung aufweist.

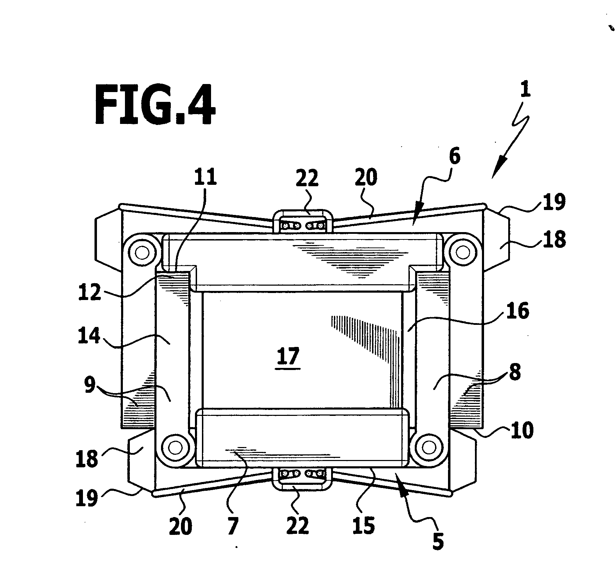

Die Schwenkachsen der beiden Anlageelemente 5, 6 haben einen Abstand voneinander, der sich zwischen den beiden Anlageelementen 5, 6 geringfügig unterscheidet, so daß die Schwenkteile 8, 9 der beiden Anlageelemente 5, 6, wenn sie rechtwinklig zu dem Stützabschnitt 7 umgeklappt sind, flächig aufeinanderliegen (Figur 4). Diese um 90° verschwenkte Position der Schwenkteile 8, 9 wird als Einführposition bezeichnet, die ausgeschwenkte Stellung, in der die Schwenkteile 8, 9 in der Ebene des Stützabschnitts 7 verlaufen, als Implantierposition.

In der Einführposition ragen die beiden Schwenkteile 8, 9 in den Zwischenraum zwischen den beiden Anlageelementen 5, 6 hinein und begrenzen diesen an seinen Längsseiten. Der übrige Zwischenraum 16 zwischen den beiden Anlageelementen 5, 6 wird bei dem dargestellten Ausführungsbeispiel von einem Kern 17 aus einem quellfähigen Material ausgefüllt, der an der Innenseite an den beiden Stützabschnitten 7 anliegt. Das Material dieses Kerns hat die Eigenschaft, bei Flüssigkeitsaufnahme das Volumen zu vergrößern. Die Volumenvergrößerung kann bis zum sechsfachen des Anfangsvolumen ohne Flüssigkeitsaufnahme gehen. Als Materialien kommen dabei prinzipiell alle nicht degradierbaren hydrophilen Polymere in Frage. Beispiele sind Polyacrylsäure und deren Derivate wie Polymethacrylsäure, Polyacrylsäureamid, Polyacrylonitril, Polyacrylsäureester, Polyhydroxyethylmethacrylate, oder auch andere Substanzen, wie beispielsweise Polyvinylpyrrolidon (PVP), Polyurethane, hochmolekularer Polyvinylalkohol.

Denkbar sind auch Polymerblends (Copolymere, welche über Bindungen miteinander verbunden sind) aus den oben genannten Polymeren oder Interpenetrating Networks (IPN) aus den oben genannten Polymeren. IPN bestehen aus mindestens zwei unterschiedlichen Polymeren, deren Polymerketten ineinander verhakt sind und über physikalische Wechselwirkungen (van der Waals-, elektrostatische, H-Brückenbindungen und / oder ionische Kräfte) miteinander verbunden sind.

Weitere Polymermischungen, welche eingesetzt werden können, sind Copolymer sowie IPN aus Polyacrylaten (Polyacrylsäure und deren Derivate wie Polymethacrylsäure, Polyacrylsäureamid, Polyacrylsäurenitril, Polyacrylsäureester) mit Polycaprolacton.

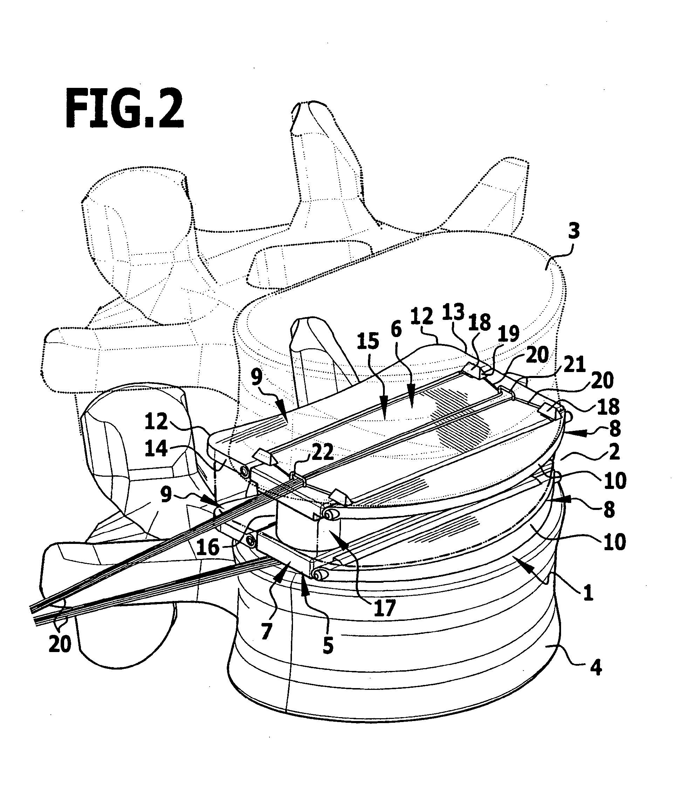

Wie aus der Darstellung der Figur 4 ersichtlich ist, ist der Querschnitt des Zwischenwirbelimplantates 1 in der Einführposition, also bei abgeklappten Schwenkteilen 8, 9, wesentlich kleiner als in der Implantierposition, in der die Schwenkteile 8, 9 in die Ebene des Stützabschnitts 7 verschwenkt sind (Figur 2).

Um diese Verschwenkbewegung durchzuführen, sind an den Schwenkteilen 8, 9 im Bereich der Schwenkachsen über die Schwenkachsen nach oben vorstehende Leisten 18 angeordnet, an deren über die Schwenkachse vorstehenden freien Enden 19 jeweils ein Zugfaden 20 angreift, der von diesen Angriffspunkten weg an der Oberseite der Stützabschnitte 7 parallel zu diesen und quer zu der Schwenkachse der Schwenkteile 8, 9 verläuft. In dem in der Zeichnung dargestellten Ausführungsbeispiel trägt jedes Schwenkteil 8 zwei derartige Leisten 18, so daß zwei Zugfäden 20 an jedem Schwenkteil 8, 9 angreifen. Alle Zugfäden 20 sind durch Umlenkösen 21, 22 an der Oberseite der Stützabschnitte 7 hindurchgeführt, die auf der Längsmittelachse der Stützabschnitte 7 angeordnet sind und die es ermöglichen, die Zugfäden 20 so umzulenken, daß sie längs der Längsmittelachse des Stützabschnitts 7 verlaufen.

Die Leisten 18 wirken außerdem auch als Anschlag, durch den die Ausschwenkbewegung der Schwenkteile 8, 9 begrenzt wird, sobald die Implantierposition erreicht ist, sobald also die Schwenkteile 8, 9 in derselben Ebene stehen wie der Stützabschnitt 7. Schließlich bilden die Leisten 18 auch Vorsprünge an der Anlagefläche aus, die sich in die Substanz der anliegenden Wirbelknochen eingraben und daher wie Rippen oder Dorne bei herkömmlichen Implantaten wirken, durch diese Vorsprünge wird die Lage des Implantates relativ zum Wirbelkörper fixiert.

Die Zugfäden 20 können beispielsweise chirurgische Nähfäden sein, vorteilhafterweise verwendet man ein Material, das im Körper resorbierbar ist. Als Material für ein solches resorbierbares Nahtmaterial kann beispielsweise verwendet werden Polyglykolsäure, Poly-p-Dioxanon, Copolymere aus Glykolsäure und/oder Trimethylenkarbonat und/oder Caprolakton und/oder P-Dioxanon und/oder Milchsäure. Diese Substanzen können in unterschiedlichen Gewichtsanteilen und in unterschiedlichsten Kombinationen eingesetzt werden.

Zum Implantieren des Zwischenwirbelimplantates 1 werden zunächst die Schwenkteile 8, 9 in die Einführposition gebracht, wie sie in Figur 4 dargestellt ist. In diesem zusammengeklappten Zustand weist das Zwischenwirbelimplantat einen relativ kleinen Querschnitt auf und kann daher auch durch kleine Zugangsöffnungen in den Körper eingeschoben werden. Bei dem Einführen verbleiben die freien Enden der Zugfäden 20 außerhalb des Körpers.

Nach dem Einführen des Zwischenwirbelimplantates kann der Operateur durch Zug an den Zugfäden 20 die Schwenkteile 8, 9 von der abgeklappten Einführposition in die ausgeschwenkte Implantierposition verschwenken, diese Bewegung erfolgt allein durch Zug an den Zugfäden 20 und gegebenenfalls durch ein Gegenhalten gegen diese Zugkräfte an den Stützabschnitten 7. Dazu kann ein geeignetes Instrument verwendet werden, das einerseits die Gegenhaltekräfte aufbringt und andererseits die Fadenenden führt.

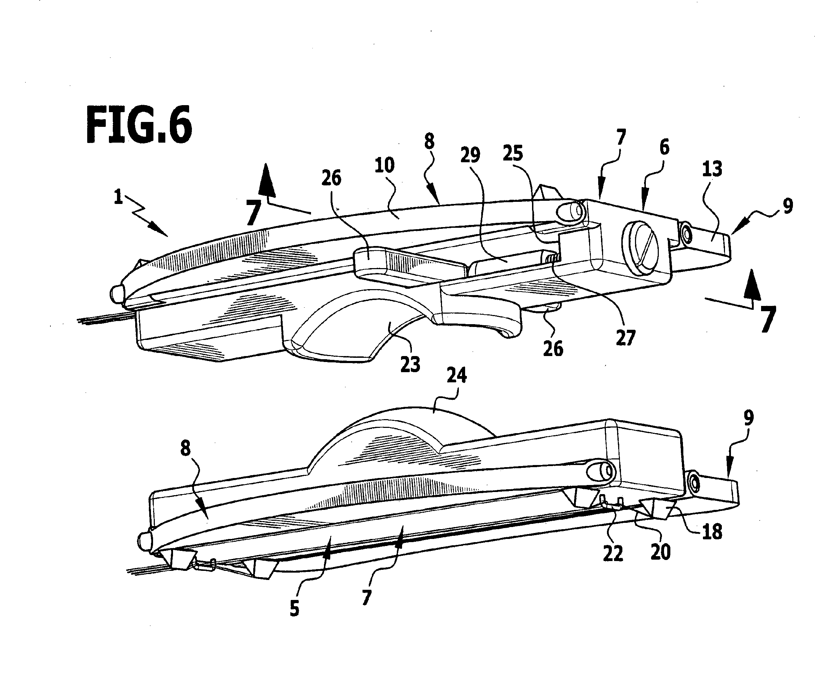

Die Schwenkteile 8, 9 müssen in der ausgeschwenkten Implantierposition festgelegt werden, damit sie ihre Stützfunktion ausüben können und nicht wieder in die abgeklappte Stellung zurückschwenken. Dies kann auf sehr unterschiedliche Weise erfolgen, wie nachstehend unter Bezugnahme auf die Figuren 6 bis 11 erläutert wird.

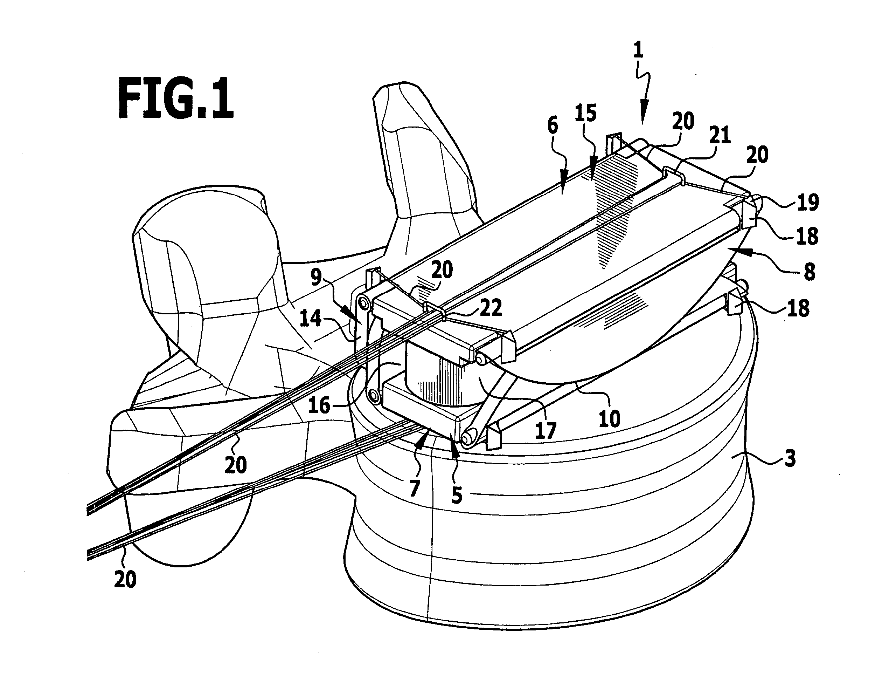

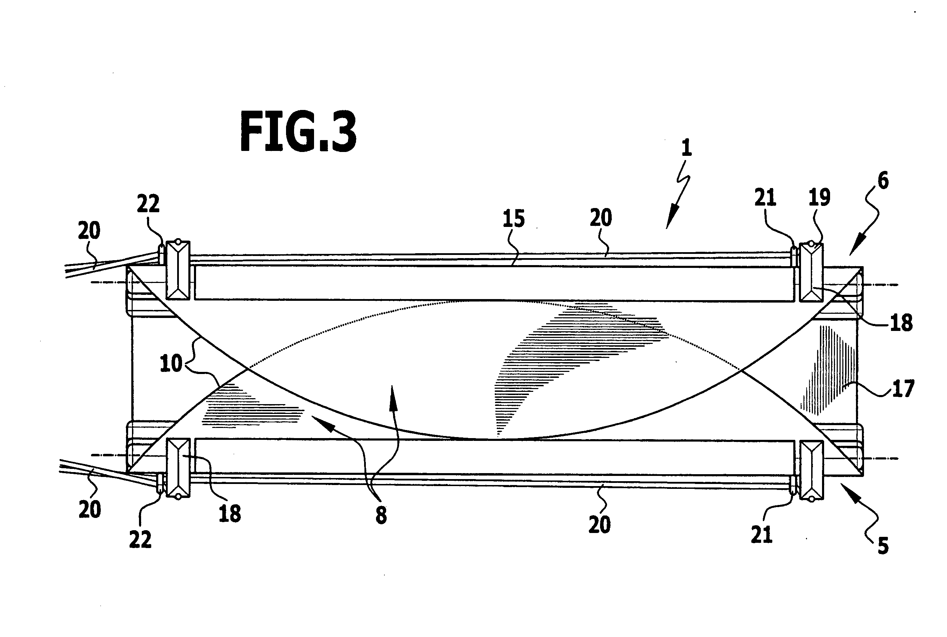

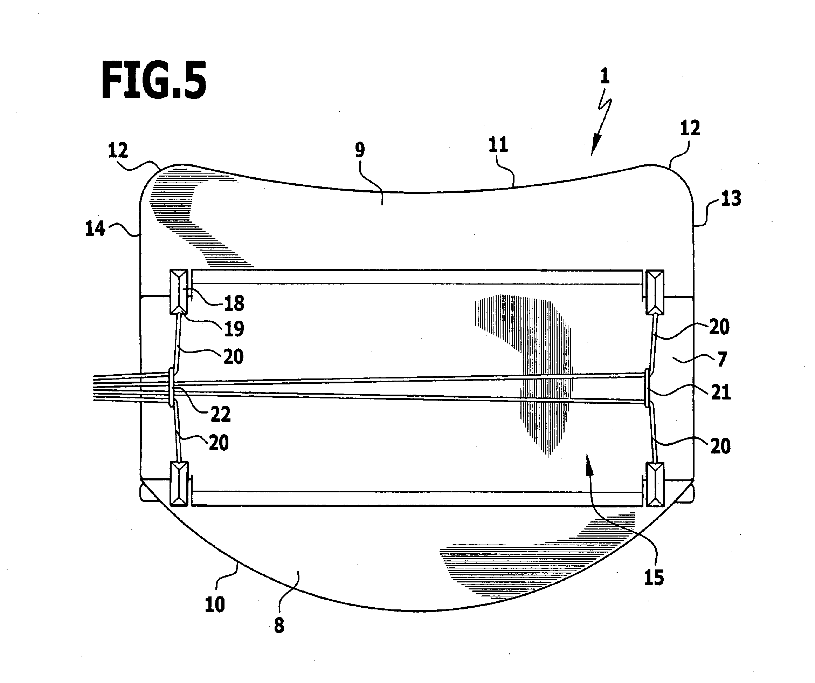

In Figur 6 ist ein Zwischenwirbelimplantat beschrieben, das nicht unter die Erfindung fällt und das sich von dem Zwischenwirbelimplantat der Figuren 1 bis 5 u.a. dadurch unterscheidet, daß das untere Anlageelement 5 und das obere Anlageelement 6 nicht über einen quellfähigen Kern 17 miteinander verbunden sind, sondern über eine ballige Lagerschale 23 und einen in diese eingreifenden, zur Lagerschale 23 komplementären Lagervorsprung 24. Dadurch sind die beiden Anlageelemente 5, 6 in geringem Umfang gegeneinander verschwenkbar, jedoch gegen eine seitliche Verschiebung gesichert. Im übrigen ist ein ähnlicher Aufbau der Anlageelemente 5, 6 gewählt. Alle Ausführungen des Zwischenwirbelimplantats können entweder mit einem quellfähigen Kern oder in der beschriebenen Weise mit einer Lagerschale und einem Lagervorsprung ausgebildet sein, grundsätzlich sind auch andere Verbindungen der beiden Anlageelemente 5, 6 möglich.

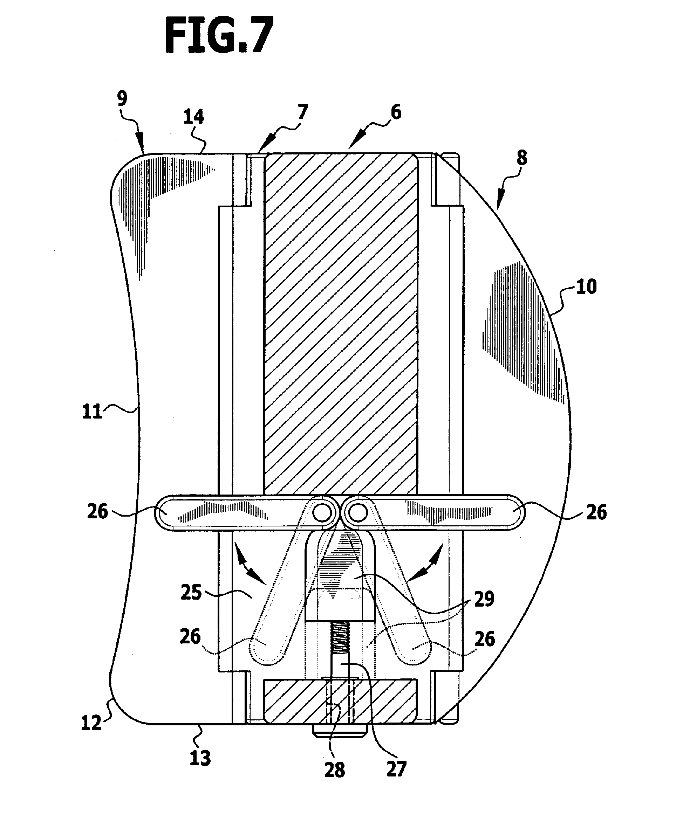

Bei dem Vergleichsbeispiel der Figur 6 sind zusätzlich in einer Aufnahmekammer 25 unterhalb des zentralen Stützabschnittes 7 zwei Hebel 26 um eine senkrecht zu den Anlageelementen 5, 6 verlaufende Drehachse verschwenkbar gelagert. In einer Einführposition sind beide Hebel 26 vollständig in die Aufnahmekammer 25 eingeschwenkt, sie können aber so aus der Aufnahmekammer 25 herausgeschwenkt werden, daß sie seitlich über die Kontur des Stützabschnittes 7 vorstehen. Zum Verschwenken der Hebel 26 ist am Stützabschnitt 7 eine Gewindespindel 27 in einer Gewindebohrung 28 verdrehbar gelagert, die an ihrem freien Ende einen Druckkörper 29 trägt, der beim Vorschieben gegen die beiden in die Aufnahmekammer 25 eingeschwenkten Hebel 26 diese ausschwenkt (Figur 7). Die Hebel 26 werden ausgeschwenkt, sobald die Schwenkteile 8, 9 die Implantierposition erreicht haben, und dann legen sich die ausgeschwenkten Hebel 26 an die Unterseite der beiden ausgeschwenkten Schwenkteile 8, 9 an und stützen diese, so daß die Schwenkteile 8, 9 nicht mehr in die abgeklappte Stellung zurückschwenken können.

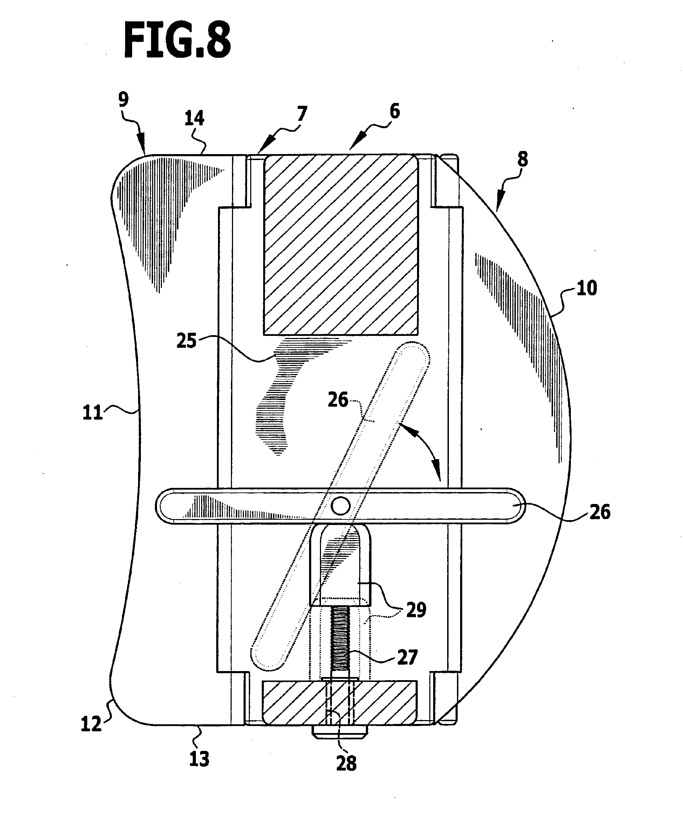

Beim Vergleichsbeispiel der Figur 8 ist ein sehr ähnlicher Aufbau gewählt, es ist lediglich statt der zwei in die Aufnahmekammer 25 einschwenkbaren Hebel 26 nur ein einziger Hebel 26 vorgesehen, der im ausgeschwenkten Zustand nach beiden Seiten aus der Aufnahmekammer 25 vorsteht und dadurch beide Schwenkteile 8, 9 gleichzeitig in der Implantierposition unterstützt.

Bei dem Vergleichsbeispiel der Figuren 6 bis 8 ist ein Verschwenken der Hebel 26 mit Hilfe der Gewindespindel 27 notwendig.

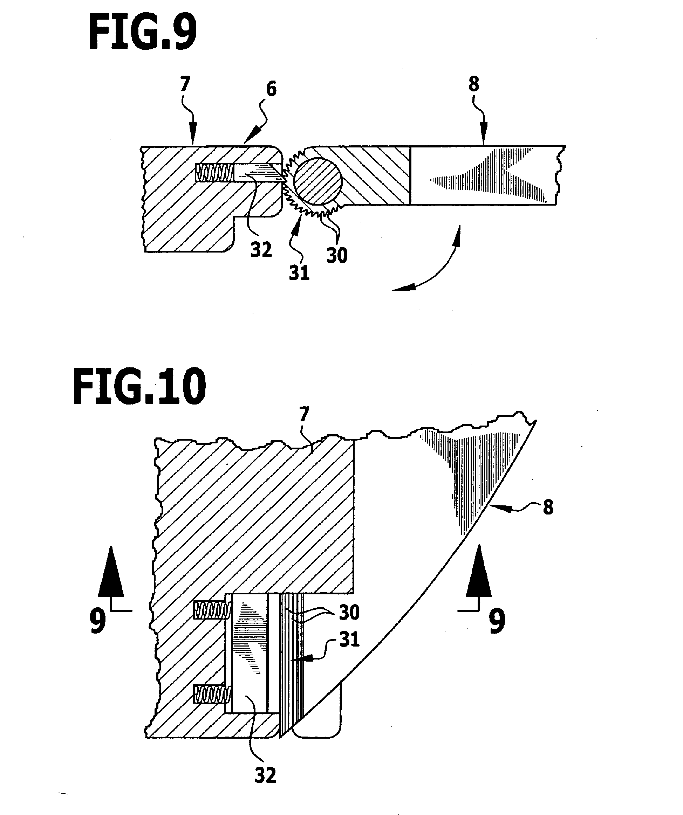

Bei den Ausführungsbeispielen der Figuren 9 bis 11 ergibt sich eine selbsttätige Verriegelung der Schwenkteile 8, 9 in der ausgeschwenkten Implantierposition. Beim Ausführungsbeispiel der Figuren 9 und 10 sind dazu die Schwenkteile im Bereich der Schwenklagerung außenseitig mit einer mit Rasten 30 versehenen Rastfläche 31 ausgestattet, und in den Stützabschnitten 7 sind quer zur Schwenkachse verschiebbare, federbelastete Rastelemente 32 gelagert, die an der Rastfläche 31 anliegen und beim Verschwenken der Schwenkteile 8, 9 an den Rastflächen 31 entlang gleiten. Dabei ist die Geometrie der Rasten 30 und der Rastelemente 32 so gewählt, daß die Teile in einer Richtung aneinander entlang gleiten können, während in der entgegengesetzten Richtung eine Verrastung durch Eingriff des Rastelementes 32 in die Rasten 30 eintritt und damit eine Verriegelung des Schwenkteils 8, 9. Mit anderen Worten kann jedes Schwenkteil 8, 9 nur aus der Einführposition in die Implantierposition verschwenkt werden, nicht aber in umgekehrter Richtung.

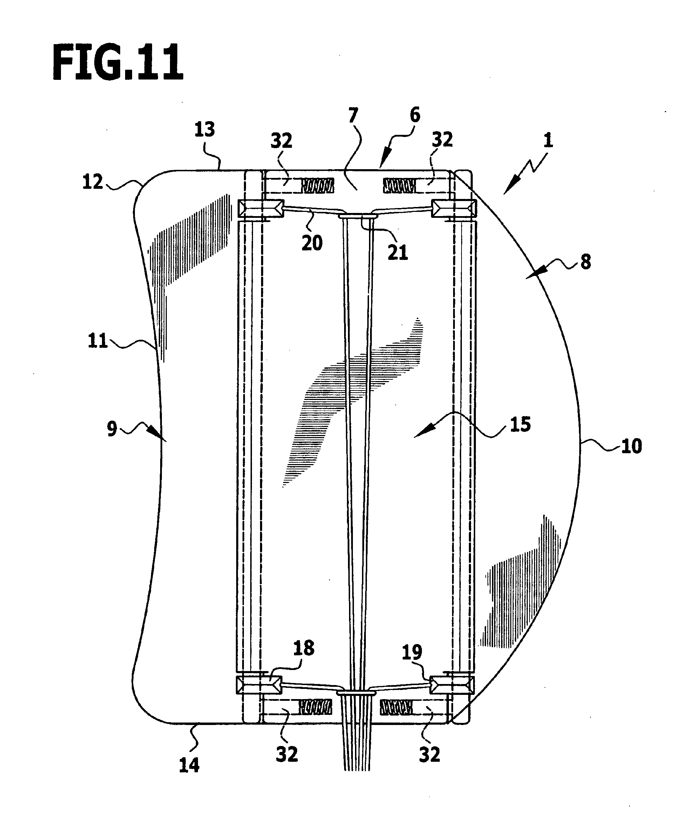

Bei dem Ausführungsbeispiel der Figur 11 sind in ähnlicher Weise stiftförmige Rastelemente 32 elastisch verschiebbar im Stützabschnitt 7 gelagert, diese greifen formschlüssig in Ausnehmungen der Schwenkteile 8, 9 ein, sobald diese die ausgeschwenkte Implantierposition erreicht haben, so daß dadurch eine Festlegung der Schwenkteile 8, 9 in der ausgeschwenkten Implantierposition erreicht wird.

Nach dem Ausschwenken der Schwenkteile 8, 9 in die Implantierposition werden die Zugfäden 20 nicht mehr benötigt, sie können abgeschnitten oder durch einen kräftigen Zug abgerissen werden. Dabei ist es vorteilhaft, wenn die Zugfäden 20 beispielsweise an den Leisten 18 nur mit einer Kraft festgehalten werden, die geringer ist als die Reißfestigkeit der Zugfäden 20, so daß bei einem kräftigen Zug an den Zugfäden 20 die Zugfäden 20 definiert an den Verbindungsstellen zu den Leisten 18 abreißen und daher vollständig entfernt werden können.

Sobald das Implantat sich im Körper befindet, kommt es mit Körperflüssigkeit in Berührung, und dies führt dazu, daß der Kern 17 durch Quellung sein Volumen vergrößert. Er füllt den Zwischenraum 16 vollständig aus und dehnt sich auch seitlich aus, dabei liegt das Material des Kerns 17 nicht nur an der Innenseite des Stützabschnitts 7 an, sondern es legt sich auch an die Unterseite der beiden Schwenkteile 8, 9 an und drückt diese dadurch in die ausgeschwenkte Implantierstellung. Damit wird eine Rastvorrichtung, die die Schwenkteile 8, 9 in der Implantierposition hält, unterstützt, gegebenenfalls kann sogar beim Fehlen einer solchen Rastvorrichtung der Kern 17 durch diese Kräfte die Schwenkteile 8, 9 dauerhaft in die Implantierposition drücken und damit gegen die Stirnflächen der beiden anliegenden Wirbelkörper 3, 4.

## Patentansprüche

1. Zwischenwirbelimplantat (1) mit mindestens einem eine Wirbelkörperanlagefläche (15) ausbildenden Anlageelement (5, 6), bei dem die Wirbelkörperanlagefläche (15) mindestens zwei relativ zueinander bewegliche Teile (8, 9) umfasst, die in einer ersten Einführposition relativ zueinander so angeordnet sind, dass sie gemeinsam einen kleinen Querschnitt einnehmen, und in einer zweiten Implantierposition so, dass der Querschnitt der Wirbelkörperanlagefiäche (15) gegenüber der Einführposition vergrößert ist, und mit einer Verstellvorrichtung zur Bewegung der beweglichen Teile (8, 9) von der Einführposition in die Implantierposition, wobei die Verstellvorrichtung mindestens ein flexibles Zugelement (20) umfasst, welches an einem der relativ zueinander beweglichen Teile (8, 9) angreift und auf Zug dieses relativ zum anderen Teil (7) in die Implantierposition bewegt, wobei auf der der Wirbelkörperanlagefläche (15) abgewandten Seite des Anlageelements (5, 6) quellfähiges Material (17) angeordnet ist, das bei Flüssigkeitsaufnahme eine Volumenvergrößerung erfährt und dadurch die Teile (8, 9) des Anlageelementes (5, 6) in die Implantierposition drückt.

2. Zwischenwirbelimplantat nach Anspruch 1, **dadurch gekennzeichnet, dass** das flexible Zugelement (20) so lang ist, dass ein Ende nach dem Einführen des Zwischenwirbelimplantates (1) in den Körper außerhalb des Körpers verbleibt, so dass über dieses extrakorporale Ende ein Zug auf das flexible Zugelement (20) ausgeübt werden kann.

3. Zwischenwirbelimplantat nach Anspruch 1 oder 2, **dadurch gekennzeichnet, dass** das flexible Zugelement (20) am anderen Teil (7) verschieblich geführt ist.

4. Zwischenwirbelimplantat nach Anspruch 3, **dadurch gekennzeichnet, dass** das flexible Zugelement (20) am anderen Teil (7) durch eine Umlenkung (21, 22) geführt ist, die die Richtung des flexible Zugelement (20) ändert.

5. Zwischenwirbelimplantat nach einem der voranstehenden Ansprüche, **dadurch gekennzeichnet, dass** das flexible Zugelement (20) lösbar mit dem durch Zug bewegbaren Teil (8, 9) verbunden ist, wobei die Lösung erst nach Überschreitung eines bestimmten Zugwertes eintritt.

6. Zwischenwirbelimplantat nach einem der voranstehenden Ansprüche, **dadurch gekennzeichnet, dass** das flexible Zugelement (20) ein chirurgischer Nähfaden ist.

7. Zwischenwirbelimplantat nach einem der voranstehenden Ansprüche, **dadurch gekennzeichnet, dass** das flexible Zugelement (20) aus einem im Körper resorbierbaren Material besteht.

8. Zwischenwirbelimplantat nach einem der voranstehenden Ansprüche, **dadurch gekennzeichnet, dass** an einem bewegbaren Teil (8, 9) mehrere Zugelemente (20) angreifen.

9. Zwischenwirbelimplantat nach einem der voranstehenden Ansprüche, **dadurch gekennzeichnet, dass** ein Anlageelement (5, 6) mehrere bewegbare Teile (8, 9) aufweist und dass jedem bewegbaren Teil (8, 9) mindestens ein Zugelement (20) zugeordnet ist.

10. Zwischenwirbelimplantat nach einem der voranstehenden Ansprüche, **dadurch gekennzeichnet, dass** eine Rastvorrichtung (30, 31, 32) vorgesehen ist, die beim Bewegen des bewegbaren Teils (8, 9) wirksam wird, sobald das bewegbare Teil (8, 9) die Implantierposition erreicht hat, und das bewegbare Teil (8, 9) gegenüber dem anderen Teil (7) fixiert.

11. Zwischenwirbelimplantat nach Anspruch 10, **dadurch gekennzeichnet, dass** die Rastvorrichtung eine mit Rasten (30) versehene Rastfläche (31) an einem der beiden Teile (7; 8, 9) und ein elastisch gegen die Rastfläche (31) gedrücktes Rastglied (32) am anderen der beiden Teile (7; 8, 9) aufweist, die beim Bewegen der bewegbaren Teile (8, 9) in die Implantierposition aneinander entlang gleiten.

12. Zwischenwirbelimplantat nach Anspruch 10, **dadurch gekennzeichnet, dass** die Rastvorrichtung an einem der beiden Teile (7; 8, 9) gelagerte, elastisch verschiebbare Rastelemente (32) umfasst, die in der Implantierposition federnd und formschlüssig in eine Ausnehmung am anderen der beiden Teile (7; 8, 9) eingreifen.

13. Zwischenwirbelimplantat nach einem der Ansprüche 1 bis 9, **dadurch gekennzeichnet, dass** an einem Teil der beiden Teile (7; 8, 9) bewegbare Stützglieder (26) angeordnet sind, die in eine das andere der beiden Teile (7; 8, 9) unterstützende Position bewegbar sind, sobald das andere der beiden Teile die Implantierposition erreicht hat.

14. Zwischenwirbelimplantat nach Anspruch 13, **dadurch gekennzeichnet, dass** das Stützglied mindestens ein drehbar an einem der beiden Teile (7; 8, 9) gelagerter Hebelarm (26) ist, der in eine das andere der beiden Teile (7; 8, 9) unterstützende Position verdrehbar ist.

15. Zwischenwirbelimplantat nach Anspruch 14, **dadurch gekennzeichnet, dass** zum Verdrehen des Hebelarms (26) eine Gewindespindel (27) an dem den Hebelarm (26) lagernden Teil (7) angeordnet ist.

16. Zwischenwirbelimplantat nach einem der voranstehenden Ansprüche, **dadurch gekennzeichnet, dass** mindestens ein Anschlag (18, 7) vorgesehen ist, der die Bewegung des bewegbaren Teils (8, 9) in die Implantierposition begrenzt.

17. Zwischenwirbelimplantat nach einem der voranstehenden Ansprüche, **dadurch gekennzeichnet, dass** das Anlageelement (5, 6) mindestens zwei plattenförmige Anlageglieder (7; 8, 9) aufweist, die relativ zueinander verschwenkbar sind und die zur Überführung in die Implantierposition auseinandergeschwenkt werden.

18. Zwischenwirbelimplantat nach einem der voranstehenden Ansprüche, **dadurch gekennzeichnet, dass** am oberen und am unteren Ende des Zwischenwirbelimplantates (1) je ein in seiner Querschnittsgröße veränderbares Anlageelement (5, 6) angeordnet ist.

19. Zwischenwirbelimplantat nach einem der voranstehenden Ansprüche, **dadurch gekennzeichnet, dass** das quellfähige Material (17) zwischen den beiden Anlageelementen (5, 6) einen quellfähigen Kern ausbildet.

## Claims

1. Intervertebral implant (1) with at least one abutment element (5, 6) forming a vertebral body abutment surface (15), in which the vertebral body abutment surface (15) comprises at least two parts (8, 9) movable relative to each other, which in a first insertion position are arranged in relation to each other such that they jointly assume a small cross section, and in a second implantation position such that the cross section of the vertebral body abutment surface (15) is increased in size in relation to the insertion position, and with an adjusting device for moving the movable parts (8, 9) from the insertion position into the implantation position, the adjusting device comprising at least one flexible pull element (20) which acts on one of the parts (8, 9) movable relative to each other and when pulled moves this relative to the other part (7) into the implantation position, wherein swellable material (17) is arranged on the side of the abutment element (5, 6) remote from the vertebral body abutment surface (15) and undergoes an increase in volume upon absorption of liquid and thereby presses the parts (8, 9) of the abutment element (5, 6) into the implantation position.

2. Intervertebral implant according to claim 1, **characterized in that** the flexible pull element (20) is of such a length that one end remains outside the body after insertion of the intervertebral implant (1) into the body, so that a pulling force can be exerted on the flexible pull element (20) via this extracorporeal end.

3. Intervertebral implant according to claim 1 or 2, **characterized in that** the flexible pull element (20) is displaceably guided on the other part (7).

4. Intervertebral implant according to claim 3, **characterized in that** the flexible pull element (20) is guided on the other part (7) through a deflection means (21, 22), which changes the direction of the flexible pull element (20).

5. Intervertebral implant according to one of the preceding claims, **characterized in that** the flexible pull element (20) is detachably connected to the part (8, 9) movable by pulling, with the detachment only occurring after a specific tension value is exceeded.

6. Intervertebral implant according to one of the preceding claims, **characterized in that** the flexible pull element (20) is a surgical suture thread.

7. Intervertebral implant according to one of the preceding claims, **characterized in that** the flexible pull element (20) is composed of a material which is resorbable in the body.

8. Intervertebral implant according to one of the preceding claims, **characterized in that** a plurality of pull elements (20) act on a movable part (8, 9).

9. Intervertebral implant according to one of the preceding claims, **characterized in that** an abutment element (5, 6) has a plurality of movable parts (8, 9), and **in that** each movable part (8, 9) has at least one pull element (20) associated with it.

10. Intervertebral implant according to one of the preceding claims, **characterized in that** a locking device (30, 31, 32) is provided, which becomes effective during movement of the movable part (8, 9) as soon as the movable part (8, 9) has reached the implantation position and secures the movable part (8, 9) in relation to the other part (7).

11. Intervertebral implant according to claim 10, **characterized in that** the locking device has a locking face (31) provided with notches (30) on one of the two parts (7; 8, 9) and a locking member (32) pressed elastically against the locking face (31) on the other of the two parts (7; 8, 9), which slide along each other during movement of the movable parts (8, 9) into the implantation position.

12. Intervertebral implant according to claim 10, **characterized in that** the locking device comprises elastically displaceable locking elements (32), which are mounted on one of the two parts (7; 8, 9) and which in the implantation position engage in a recess on the other of the two parts (7; 8, 9) in a resilient and positively locking manner.

13. Intervertebral implant according to one of claims 1 to 9, **characterized in that** movable support members (26) are arranged on one part of the two parts (7; 8, 9) and are movable into a position supporting the other of the two parts (7; 8, 9) as soon as the other of the two parts has reached the implantation position.

14. Intervertebral implant according to claim 13, **characterized in that** the support member is at least one lever arm (26), which is rotatably mounted on one of the two parts (7; 8, 9) and which is rotatable into a position supporting the other of the two parts (7; 8, 9).

15. Intervertebral implant according to claim 14, **characterized in that** for rotation of the lever arm (26) a threaded spindle (27) is arranged on the part (7) supporting the lever arm (26).

16. Intervertebral implant according to one of the preceding claims, **characterized in that** at least one stop (18, 7) is provided, which restricts the movement of the movable part (8, 9) into the implantation position.

17. Intervertebral implant according to one of the preceding claims, **characterized in that** the abutment element (5, 6) has at least two plate-like abutment members (7; 8, 9), which can be pivoted relative to each other and can be pivoted apart for transition into the implantation position.

18. Intervertebral implant according to one of the preceding claims, **characterized in that** an abutment element (5, 6), which is changeable in its cross-sectional size, is arranged respectively on the upper and on the lower end of the intervertebral implant (1).

19. Intervertebral implant according to one of the preceding claims, **characterized in that** the swellable material (17) forms a swellable core between the two abutment elements (5, 6).

## Revendications

1. Implant intervertébral (1) avec au moins un élément d'appui (5, 6) formant une surface d'appui (15) de corps vertébral, où la surface d'appui (15) de corps vertébral comprend au moins deux pièces (8, 9) déplaçables l'une par rapport à l'autre, qui, dans une première position d'insertion, sont disposées l'une par rapport à l'autre de manière à occuper ensemble une section réduite, et de manière à agrandir la section de la surface d'appui (15) de corps vertébral par rapport à la position d'insertion dans une deuxième position d'implantation, et avec un dispositif d'ajustement pour le déplacement des pièces mobiles (8, 9) de la position d'insertion vers la position d'implantation, le dispositif d'ajustement comprenant au moins un élément de traction flexible (20) ayant prise sur une des pièces (8, 9) déplaçables l'une par rapport à l'autre et déplaçant celle-ci par traction par rapport à l'autre pièce (7) vers la position d'implantation, un matériau expansible (17) étant disposé sur la face de l'élément d'appui (5, 6) distante de la surface d'appui (15) de corps vertébral, dont le volume grossit par absorption de liquide et qui repousse ainsi les pièces (8, 9) de l'élément d'appui (5, 6) vers la position d'implantation.

2. Implant intervertébral selon la revendication 1, **caractérisé en ce que** la longueur de l'élément de traction flexible (20) est telle qu'une extrémité reste extérieure au corps après insertion de l'implant intervertébral (1) dans le corps, de manière à pouvoir exercer une traction sur l'élément de traction flexible (20) au moyen de ladite extrémité extracorporelle.

3. Implant intervertébral selon la revendication 1 ou 2, **caractérisé en ce que** l'élément de traction flexible (20) est guidé de manière à pouvoir coulisser sur l'autre pièce (7).

4. Implant intervertébral selon la revendication 3, **caractérisé en ce que** l'élément de traction flexible (20) est guidé par un renvoi (21, 22) sur l'autre pièce (7), lequel change la direction de l'élément de traction flexible (20).

5. Implant intervertébral selon l'une des revendications précédentes, **caractérisé en ce que** l'élément de traction flexible (20) est raccordé de manière amovible à la pièce (8, 9) déplaçable par traction, la désolidarisation ne survenant qu'au dépassement d'une valeur de traction définie.

6. Implant intervertébral selon l'une des revendications précédentes, **caractérisé en ce que** l'élément de traction flexible (20) est un fil chirurgical.

7. Implant intervertébral selon l'une des revendications précédentes, **caractérisé en ce que** l'élément de traction flexible (20) est constitué d'un matériau résorbable dans le corps.

8. Implant intervertébral selon l'une des revendications précédentes, **caractérisé en ce que** plusieurs éléments de traction (20) ont prise sur une pièce mobile (8, 9).

9. Implant intervertébral selon l'une des revendications précédentes, **caractérisé en ce qu'**un élément d'appui (5, 6) comprend plusieurs pièces mobiles (8, 9) et **en ce qu'**au moins un élément de traction (20) est associé à chaque pièce mobile (8, 9).

10. Implant intervertébral selon l'une des revendications précédentes, **caractérisé en ce qu'**il est prévu un mécanisme d'enclenchement (30, 31, 32) entrant en action lors du déplacement de la pièce mobile (8, 9) dès que la pièce mobile (8, 9) atteint la position d'implantation, et fixant la pièce mobile (8, 9) relativement à l'autre pièce (7).

11. Implant intervertébral selon la revendication 10, **caractérisé en ce que** le mécanisme d'enclenchement comporte une surface d'enclenchement (31) pourvues de crans (30) sur une des deux pièces (7 ; 8, 9) et un élément d'enclenchement (32) serré par force élastique contre la surface d'enclenchement (31) sur l'autre des deux pièces (7 ; 8, 9), lesquels glissent l'une contre l'autre lors du déplacement des pièces mobiles (8, 9) vers la position d'implantation.

12. Implant intervertébral selon la revendication 10, **caractérisé en ce que** le mécanisme d'enclenchement comprend des éléments d'enclenchement (32) déplaçables par force élastique, montés sur une des deux pièces (7 ; 8, 9), lesquels s'engagent élastiquement et par correspondance de forme dans un évidement sur l'autre des deux pièces (7; 8, 9) en position d'implantation.

13. Implant intervertébral selon l'une des revendications 1 à 9, **caractérisé en ce que** des éléments d'appui mobiles (26) sont disposés sur une pièce des deux pièces (7 ; 8, 9), lesquels sont déplaçables vers une position de soutien de l'autre des deux pièces (7 ; 8, 9) dès que l'autre des deux pièces atteint la position d'implantation.

14. Implant intervertébral selon la revendication 13, **caractérisé en ce que** l'élément d'appui est au moins un bras de levier (26) monté de manière rotative sur une des deux pièces (7 ; 8, 9), lequel est rotatif vers une position de soutien de l'autre des deux pièces (7 ; 8, 9).

15. Implant intervertébral selon la revendication 14, **caractérisé en ce que**, pour la rotation du bras de levier (26), une vis filetée (27) est disposée sur la pièce (7) où est monté le bras de levier (26).

16. Implant intervertébral selon l'une des revendications précédentes, **caractérisé en ce qu'**au moins une butée (18, 7) est prévue, laquelle limite le déplacement de la pièce mobile (8, 9) vers la position d'implantation.

17. Implant intervertébral selon l'une des revendications précédentes, **caractérisé en ce que** l'élément d'appui (5, 6) comporte au moins deux éléments de butée (7 ; 8, 9) en forme de plaque, pouvant pivoter l'un par rapport à l'autre et qui sont écartés pour aller vers la position d'implantation.

18. Implant intervertébral selon l'une des revendications précédentes, **caractérisé en ce qu'**un élément d'appui (5, 6) à section de grandeur variable est disposé à l'extrémité supérieure et à l'extrémité inférieure de l'implant intervertébral (1).

19. Implant intervertébral selon l'une des revendications précédentes, **caractérisé en ce que** le matériau expansible (17) forme un noyau expansible entre les deux éléments d'appui (5, 6).
